# EUROPÄISCHE PATENTSCHRIFT

(11) **EP 4 192 804 B1**
(45) Veröffentlichungstag und Bekanntmachung des Hinweises auf die Patenterteilung: **30.10.2024**
(21) Anmeldenummer: 21735572.6
(22) Anmeldetag: 25.06.2021
(51) Int. Cl.: C07C 29/152, C07C 31/04, B01D 53/14

(54) **VORRICHTUNG UND VERFAHREN ZUR HERSTELLUNG VON METHANOL AUS KOHLENDIOXID**
DEVICE AND METHOD FOR PRODUCING METHANOL FROM CARBON DIOXIDE
DISPOSITIF ET PROCÉDÉ DE PRODUCTION DE MÉTHANOL À PARTIR DE DIOXYDE DE CARBONE

(30) Priorität: 07.08.2020 DE 102020120879
(43) Veröffentlichungstag der Anmeldung: 14.06.2023
(73) Patentinhaber: Karlsruher Institut für Technologie, 76131 Karlsruhe (DE)
(72) Erfinder: VIDAL-VAZQUEZ, Francisco, 68165 Mannheim (DE); DITTMEYER, Roland, 76133 Karlsruhe (DE); PFEIFER, Peter, 76351 Linkenheim-Hochstetten (DE)
(86) Internationale Anmeldenummer: PCT/EP2021/025234
(87) Internationale Veröffentlichungsnummer: WO 2022/028730

(56) Entgegenhaltungen:
- CN-A- 111 302 896
- DE-A1- 102017 202 313

## Beschreibung

Die Erfindung betrifft eine Vorrichtung sowie ein Verfahren zur Herstellung von Methanol aus Kohlendioxid gemäß dem ersten bzw. dem dreizehnten Patentanspruch.

Als ein Hauptverursacher des globalen Klimawandels gilt die Emission von Treibhausgasen wie Kohlendioxid, CO₂, aus technischen Prozessen wie z.B. Verbrennungsprozessen oder bei der Herstellung von z.B. Zement oder Stahl in die Erdatmosphäre. Ein möglicher Ansatz zur Reduktion von CO₂-Emissionen stellt die Reduzierung des Einsatzes fossiler Brennstoffe zugunsten eines erhöhten Einsatzes erneuerbarer Energien dar. Erneuerbare Energien liegen jedoch oftmals als elektrische Energie vor, und nur diese stellt damit insbesondere bei energieintensiven Prozessen wie z.B. der Herstellung von Zement oder Stahl oder bei der Langstreckenmobilität auch langfristig keine Alternative zu chemischen Energieträgern dar.

Ein anderer Ansatz für eine Emissionsreduzierung in der Kohlendioxidbilanz bieten die sog. CCU-Technologien (Carbon Capture and Utilization). Sie entziehen entstehendes CO₂ aus den Emissionen, insbesondere von Verbrennungsabgasen, oder der Atmosphäre und wandeln dieses mit erneuerbarer Energie in chemischen Prozessen in kohlenstoffhaltige Kraftstoffe und Chemikalien um.

Die Herstellung von Methanol gilt aufgrund dessen Relevanz als Grundchemikalie als eine der wichtigsten CCU Anwendungen. Mit einer weltweiten Produktion von über 110 Millionen Tonnen pro Jahr wird Methanol zur Herstellung von Produkten, chemischen Zwischenprodukten, Kunststoffen und Feinchemikalien benötigt. Bekannt sind darüber hinaus auch Verfahren zur Umwandlung von Methanol in kohlenwasserstoffhaltige Kraftstoffe oder auch in die Nutzung von Methanol selbst als Kraftstoff.

Aus der WO 2001/17936 A2 ist beispielsweise ein Verfahren und eine Anlage zur Synthese von Methanol aus einem Gasgemisch aus Wasserstoff, Kohlenmonoxid und Kohlendioxid bekannt. Das Gasgemisch wird unter Druck in wenigstens einer Synthesestufe zu Methanol umgesetzt, wobei das für den Einsatz in einem katalysatorgefüllten Methanolsynthesereaktor bestimmte Gasgemisch direkt vor Zugabe in besagten Methanolsynthesereaktor in einem zusätzlichen Trimmerhitzer aufgeheizt wird. Das angestrebte Ziel der Vorwärmung lag bei diesem Stand der Technik in der Einsparung von Wärmetauscherfläche und/oder die Erhöhung der Ausbeute unter besondere Berücksichtigung der veränderlichen Katalysatoraktivität.

DE 10 2017 202 313 A1 offenbart ferner eine Vorrichtung und ein Verfahren zur Herstellung von chemischen Wertstoffen aus Kohlendioxid und Wasserstoff als Edukte. Kohlendioxid wird mit einem Rohgas mit Hilfe eines Absorbers und Desorbers entnommen, wobei sich der Kreislauf im Wärmetauscher kreuzt. Der für die Synthese benötigte Wasserstoff wird dem Desorber zugeführt, um Kohlendioxid aus den beladenen Absorptionsmitteln auszutreiben. Kohlendioxid und Wasserstoff werden dann im Synthesereaktor zum Wertstoff umgesetzt. Gemäß einer vorteilhaften Ausgestaltung kann das Absorptionsmittel ein physikalisches Absorptionsmittel, insbesondere Methanol, Wasser, Polyethylenglykoldiemthylether oder Pyrrolidone sein.

Davon ausgehend liegt eine **Aufgabe der Erfindung** darin, eine Vorrichtung zur Herstellung von Methanol aus Kohlendioxid vorzuschlagen, die einen gesamten Methanolsyntheseprozess von der Gewinnung von Kohlendioxid aus Gasmischungen bis hin zur Erzeugung eines Methanolstroms abbildet.

Weiterhin soll ein Verfahren zur Herstellung von Methanol aus Kohlendioxid mit diesem gesamten Methanolsyntheseprozess vorgeschlagen werden.

Die Aufgabe wird mit einer Vorrichtung und einem Verfahren gemäß dem Anspruch 1 bzw. 13 gelöst. Hierauf rückbezogene Unteransprüche geben vorteilhafte Ausführungsformen wieder.

Die Lösung der Aufgabe umfasst eine Vorrichtung und ein Verfahren zur Herstellung von Methanol aus einem CO2-haltigen Gasgemisch wie einem Verbrennungsabgas oder einem Prozessgas sowie Wasserstoff.

Eine vorgeschlagene **Vorrichtung** zur Herstellung von Methanol aus Kohlendioxid, umfasst drei Kreislaufprozesse.

Der **erste Kreislaufprozess** mit einer ersten umlaufenden Leitung für ein umlaufendes erstes Gemisch aus Wasser und Methanol, umfasst:
i) eine Absorptionsstufe für Kohlendioxid mit einem Durchlass für ein kohlendioxidhaltiges Gas,
ii) eine Desorptionsstufe für Kohlendioxid mit einem Zulauf für Wasserstoff,
iii)einem ersten Wärmetauscher zwischen der Absorptionsstufe und der Desorptionsstufe, wobei sich die umlaufende Leitung im Wärmetauscher kreuzt,
iv) einen Auslass für das erste Gemisch aus Wasser und Methanol zwischen der Desorptionsstufe und dem ersten Wärmetauscher,
v) eine Umwälzpumpe zwischen der Absorptionsstufe und der Desorptionsstufe sowie
vi) eine Expansionsdrossel zwischen dem ersten Wärmetauscher und der Absorptionsstufe.

Der **zweite Kreislaufprozess** mit einer zweiten umlaufenden Leitung für ein umlaufendes zweites Gemisch aus Methanol, Wasser, Kohlendioxid und Wasserstoff, umfasst:
i) eine Desorptionsstufe für Kohlendioxid mit dem Zulauf für Wasserstoff sowie
ii) eine erste Flüssigkeits-Gas-Phasentrennstufe mit einem Rücklauf für flüssige Phasen des Methanols und des Wassers zu der Desorptionsstufe als Teil der zweiten umlaufenden Leitung sowie einem Gasauslass und einem Einlass für ein Flüssigkeits-Gas-Phasengemisch aus der Desorptionsstufe.

Der **dritte Kreislaufprozess** mit einer dritten umlaufenden Leitung für ein umlaufendes drittes Gemisch aus Kohlendioxid und Wasserstoff, umfasst:
i) einen Methanolsynthesereaktor mit einer Kühlung,
ii) die erste Flüssigkeits-Gas-Phasentrennstufe, wobei der Gasauslass in die dritte umlaufende Leitung ausmündet und die dritte umlaufende Leitung in den Einlass einmündet,
iii)einen zweiten Wärmetauscher zwischen dem Methanolsynthesereaktor und der ersten Flüssigkeits-Gas-Phasentrennstufe, wobei sich die dritte umlaufende Leitung in dem zweiten Wärmetauscher kreuzt,
iv) einen Gasauslass in der dritten umlaufenden Leitung zwischen dem Methanolsynthesereaktor und dem zweiten Wärmetauscher,
v) ein Gebläse zwischen dem zweiten Wärmetauscher und dem Einlass.

Ein vorgeschlagenes **Verfahren** zur Herstellung von Methanol aus Kohlendioxid, umfasst zumindest folgende Verfahrensschritte:
a) Bereitstellung einer Vorrichtung zur Herstellung von Methanol aus Kohlendioxid, wie zuvor beschrieben, einschließlich optionaler Ausgestaltungen,
b) Durchleitung eines kohlendioxidhaltigen Gases durch die Absorptionsstufe, wobei das Kohlendioxid bei einem Druck zwischen 1 bar und 10 bar (Grenzwerte jeweils eingeschlossen) und einer Temperatur zwischen -30°C und 10°C (Grenzwerte jeweils eingeschlossen) von einem flüssigen ersten Gemisch aus Wasser und Methanol in den ersten Kreislaufprozess aufgenommen wird,
c) Komprimierung und Temperierung des ersten Gemisches mit dem Kohlendioxid in der Umwälzpumpe bzw. im ersten Wärmetauscher des ersten Kreislaufprozesses auf einen Druck zwischen 40 bar und 50 bar (Grenzwerte jeweils eingeschlossen) und eine Temperatur zwischen 100°C und 180°C (Grenzwerte jeweils eingeschlossen),
d) Einleitung des komprimierten und temperierten ersten Gemisches mit dem Kohlendioxid in die Desorptionsstufe, wobei das Kohlendioxid aus dem ersten Gemisch desorbiert und dem ersten Kreislaufprozess entnommen wird und unter Hinzufügung von Wasserstoff von einem umlaufenden zweiten Gemisch aus Methanol, Wasser, Kohlendioxid und Wasserstoff im zweiten Kreislaufprozess bei einem Druck zwischen 40 bar und 50 bar (Grenzwerte jeweils eingeschlossen) und einer Temperatur zwischen 100°C und 180°C (Grenzwerte jeweils eingeschlossen) aufgenommen wird,
e) Weiterleitung des Kohlendioxids im zweiten Kreislaufprozess in die erste Flüssigkeits-Gas-Phasentrennstufe, in der ein Gasgemisch aus Kohlendioxid und Wasserstoff selektiv als drittes Gemisch vom zweiten in den dritten Kreislaufprozess umgeleitet wird,
f) Aufwärmung des Gasgemisches im zweiten Wärmetauscher im dritten Kreislaufprozess auf eine Temperatur zwischen 210°C und 260°C (Grenzwerte jeweils eingeschlossen),
g) Einleitung des temperierten Gasgemisches in den Methanolsynthesereaktor und partielle Umsetzung des dritten Gemisches zu Methanol und Wasser,
h) Zurückleitung des dritten Gemisches, des Methanols und des Wassers durch den zweiten Wärmetauscher hindurch in die erste Flüssigkeits-Gas-Phasentrennstufe,
i) Abtrennung der flüssigen Bestandteile des Gemisches, des Methanols und des Wassers in der ersten Flüssigkeits-Gas-Phasentrennstufe und Rückführung dieser über den zweiten Kreislaufprozess in die Desorptionsstufe sowie
j) Kontinuierliche Abzweigung eines Gemisches aus Wasser und Methanol aus der Desorptionsstufe.

Ein Grundgedanke der Lösung besteht darin, die Abscheidung von Kohlendioxid aus einem Abgas oder einem anderen kohlendioxidhaltigen Gas in die Kreislaufprozesse der Methanolsyntheseprozess zu integrieren. Für die Abtrennung von Kohlendioxid wird mit einem Gemisch aus Methanol und Wasser ein flüssiges Absorptionsmittel herangezogen, das im Rahmen der nachfolgenden Methanolsynthese bereits vorliegt. Dabei wird auch vorgeschlagen das sich in der Methanolsynthese bildende Methanol zusammen mit Wasser zu kondensieren und zumindest partiell der Abscheidung des Kohlendioxids aus dem Abgas oder anderen kohlendioxidhaltigen Gasen zur Verfügung zu stellen.

Ein weiterer Grundgedanke der Lösung liegt darin, das in das Gemisch aus Methanol und Wasser aufgenommene Kohlendioxid mit Wasserstoff als Spülgas wieder aus dem Gemisch zu desorbieren und es dann gemeinsam mit dem Wasserstoff der Methanolsynthese zuzuführen.

Ferner wird vorzugsweise vorgeschlagen, die Methanolsynthese und die Desorption des Kohlendioxids aus dem Gemisch aus Methanol und Wasser auf demselben Druckniveau durchzuführen.

In einem technischen Prozess wie z.B. einem Verbrennungsprozess oder einem Herstellprozess von z.B. Zement oder Stahl integriert liegt ein weiterer Vorteil nicht nur in der Reduzierung des CO₂-Ausstoßes des technischen Prozesses, sondern auch in der direkten Einbindung einer Wasserstoffelektrolyse für die Bereitstellung des vorgenannten Spülgases im Gesamtprozess. Damit reduzieren sich in vorteilhafter Weise im Vergleich zu dem herkömmlichen Verfahren zur Methanolsynthese in Kombination mit einer CO₂-Abscheidung auch die Kosten für Vorrichtung und Betrieb.

Der besondere Vorteil der Erfindung liegt darin, dass bei niedrigen Drücken kontinuierlich Kohlendioxid aus einem Rauchgasstrom oder einem Prozessgasstrom unabhängig vom jeweiligen Kohlendioxidgehalt (typischerweise 10 bis 90 Vol.%) abgetrennt wird. Diese Rauchgasströme oder Prozessgasströme entstammen aus industriellen und nichtindustriellen Prozessen wie beispielsweise:
- Biogas mit Kohlenstoffkonzentrationen typischerweise von 40-50 Vol.-%,
- Rauchgas aus einem industriellen Prozess wie einem Kraftwerk, einem chemischen Prozess, der Stahlherstellung, der Zementherstellung und dergleichen
- Rauchgas aus einer Müllverbrennungsanlage.
- Kohlendioxidhaltiges Synthesegas aus der Biomassevergasung.

Die Erfindung wird anhand von Ausführungsbeispielen, den folgenden Figuren und Beschreibungen näher erläutert. Alle dargestellten Merkmale und deren Kombinationen sind nicht nur auf diese Ausführungsbeispiele und deren Ausgestaltungen begrenzt. Vielmehr sollen diese stellvertretend für weitere mögliche, aber nicht explizit als Ausführungsbeispiele dargestellte weitere Ausgestaltungen kombinierbar angesehen werden. Es zeigen
**Fig.1** schematisch ein erstes Ausführungsbeispiel einer Vorrichtung zur Herstellung von Methanol aus Kohlendioxid, umfassend drei Kreislaufprozesse,
**Fig.2** schematisch das erste Ausführungsbeispiel gemäß **Fig.1****,** jedoch mit zusätzlichen Temperierungsmitteln für das Verbrennungsabgas oder das Prozessgas sowie einem zusätzlichen Flüssigphasenrücklauf zu der Desorptionsstufe,
**Fig.3** schematisch ein zweites Ausführungsbeispiel einer Vorrichtung zur Herstellung von Methanol aus Kohlendioxid, umfassend drei Kreislaufprozesse sowie
**Fig.4** schematisch ein Fließbild (Prozess-FLOW-Diagramm) des Verfahrens in einer Vorrichtung gemäß der in **Fig.1** dargestellten Ausführungsform.

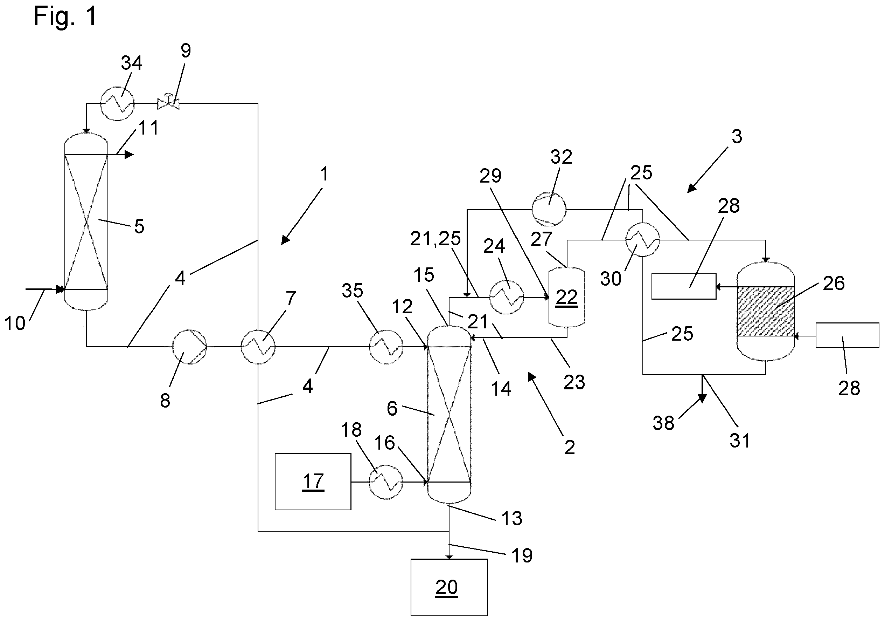

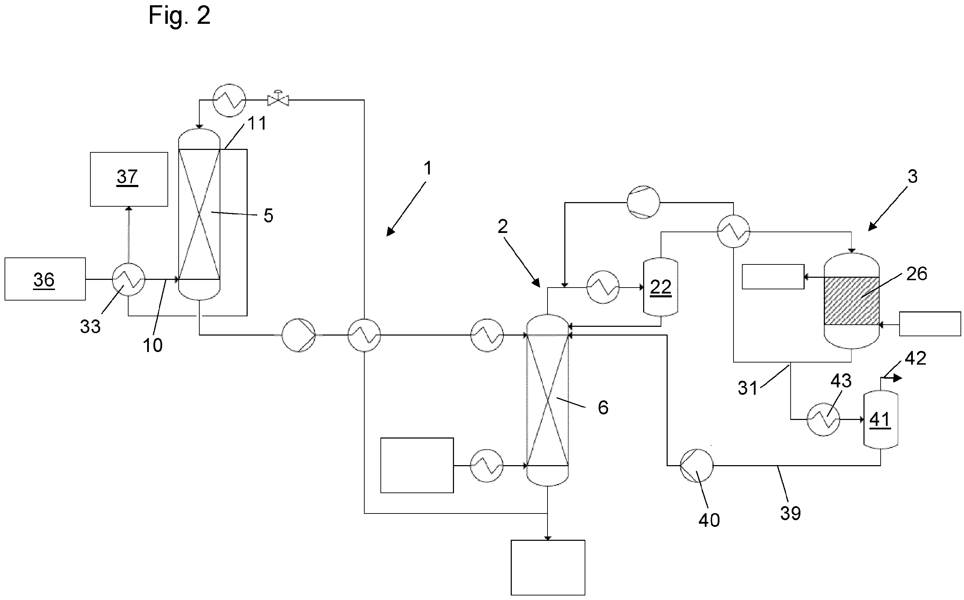

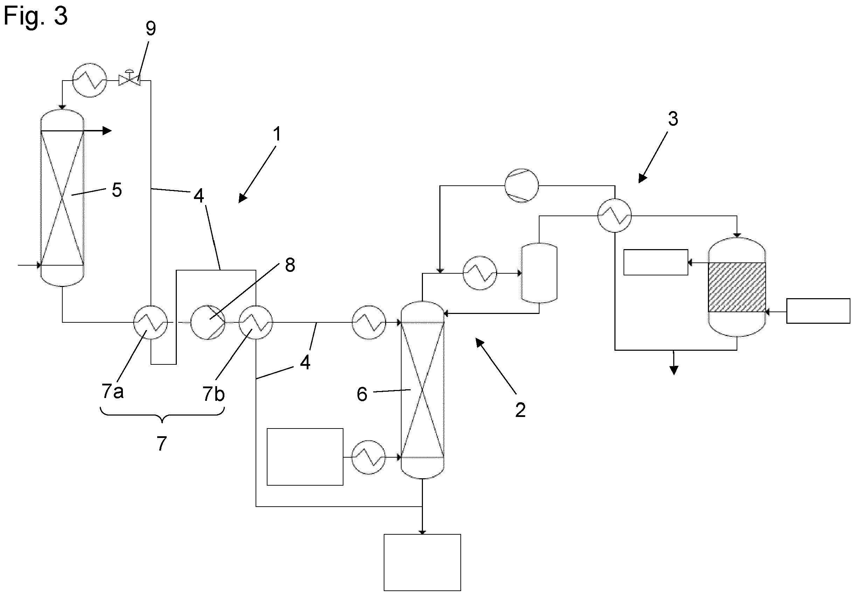

Die in **Fig.1 bis 3** dargestellten Vorrichtungen zur Herstellung von Methanol aus Kohlendioxid umfassen zumindest drei Kreislaufprozesse **1, 2** und **3.** Die dargestellten Ausführungsformen werden beispielhaft nachfolgend anhand **Fig.1** dargestellt, während optionale Ausgestaltungen auch anhand der **Fig.2** und **3** näher erläutert werden.

Ein **erster Kreislaufprozess 1** weist eine erste umlaufende Leitung **4** für ein umlaufendes erstes Gemisch aus Wasser und Methanol auf. In diesem ist eine Absorptionsstufe **5** sowie eine Desorptionsstufe **6** für Kohlendioxid, mindestens ein erster Wärmetauscher **7,** eine Umwälzpumpe **8** sowie eine Expansionsdrossel **9** vorgesehen. Die umlaufende Leitung kreuzt sich zwischen der Absorptionsstufe sowie eine Desorptionsstufe im Wärmetauscher.

Die umlaufende Leitung **4** des ersten Kreislaufprozesses durchläuft die Absorptionsstufe **5,** vorzugsweise von oben nach unten, gefolgt von einer seriellen Anordnung von Umwälzpumpe **8** und erstem Wärmetauscher **7,** bevor sie in die Desorptionsstufe **6** ausmündet. Die Desorptionsstufe wird wie die Absorptionsstufe dabei vorzugsweise von oben nach unten durchdrungen, gefolgt von einem Durchlauf durch den ersten Wärmetauscher **7** und einer Expansionsdrossel **9** zurück zur vorgenannten Absorptionsstufe. Die Durchleitungen durch die Absorptionsstufe und die Desorptionsstufe sind Teil der umlaufenden Leitung des ersten Kreislaufprozesses. Diese kreuzt sich zudem im ersten Wärmetauscher, geeignet für eine Wärmeübertragung zwischen den sich kreuzenden Fluidströmen des ersten Kreislaufprozesses. Die Umwälzpumpe dient der Komprimierung, die Expansionsdrossel einer entsprechenden Entspannung des umlaufenden ersten Gemisches aus Wasser und Methanol, sodass sowohl der Druck als auch die Temperatur in der Absorptionsstufe über denen in der Desorptionsstufe liegen. In vorteilhafter Weise wird eine Komprimierung von gasförmigem Kohlendioxid vermieden.

Die **Absorptionsstufe 5** weist einen Durchlass und damit mindestens einen Einlass **10** und einen Auslass **11** für ein kohlendioxidhaltiges Gas wie z.B. ein vorgenanntes Verbrennungs- oder Prozessgas auf. Der Einlass ist vorzugsweise wie dargestellt unten, der Auslass oben angeordnet. Sie ist vorzugsweise als Nasswäscher ausgestaltet, d.h. sie umfasst ein Innenvolumen, bei dem die Durchströmung mit dem kohlendioxidhaltigen Gas vorzugsweise von unten nach oben erfolgt. Das vorgenannte erste Gemisch aus Wasser und Methanol wird dagegen oben in das Innenvolumen eingebracht und durchdringt jenes im Gegenstrom zur Durchströmung nach unten und wird anschließend über einen Auslass unten wieder aus dem Volumen ausgeleitet. Das erste Gemisch ist flüssig und dient einerseits als Lösungsmittel für eine selektive Absorption von Kohlendioxid aus dem kohlendioxidhaltigen Gas, andererseits als temperaturbestimmendes Element für die Prozessbedingungen in der Absorptionsstufe.

Die **Desorptionsstufe 6** ist dagegen nicht nur in den ersten Kreislaufprozess **1,** sondern auch in den nachfolgend beschriebenen zweiten Kreislaufprozess **2** eingebunden. Dazu weist die Desorptionsstufe vorzugsweise ein, weiter bevorzugt nur ein Volumen auf, das als Mischkammer in den ersten und zweiten Kreislaufprozess gleichermaßen integriert ist. Der Zulauf **12** des ersten Kreislaufprozesses ist vorzugsweise oben, der Ablauf **13** unten im Volumen, sodass hier ein Durchlauf wie in einem Nasswäscher realisierbar ist. Einlass **14** für flüssige Bestandteile (und ggf. noch vorhandenen, d.h. in der nachfolgend beschriebenen ersten Flüssigkeits-Gas-Phasentrennstufe nicht zuvor abgetrennte Reste an Gasen) und Gasauslass **15** für die gasförmigen Bestandteile des zweiten Kreislaufprozesses ist im Volumen vorzugsweise oben, während die flüssigen Bestandteile des zweiten Kreislaufprozesses im Volumen vom ersten Kreislaufprozess aufgenommen werden und mit diesem unten im Volumen über den Ablauf **13** des ersten Kreislaufprozesses ausgeleitet werden.

Ferner weist das Volumen einen Zulauf für Wasserstoff **16,** vorzugsweise im unteren Bereich des Volumens zur Anbindung und damit Desorption des im ersten Kreislaufprozess gebundenen Kohlendioxids auf.

Der Wasserstoffeintrag erfolgt vorzugsweise direkt aus einer Hochdruckelektrolyse (Druck wird durch Pumpen von Wasser erzeugt, anstatt erzeugtes H₂ zu komprimieren) als Wasserstoffquelle **17,** wobei erneuerbarer Strom nutzbar ist. Alternativ ist der Wasserstoff aus industriellen Prozessen heranziehbar, sofern der Wasserstoff in entsprechend hoher Konzentration (vorzugsweise über 98 Vol.-%) vorliegt. Zwischen der Hochdruckelektrolyse und dem Zulauf für Wasserstoff ist zudem Wasserstofftempierungsmitttel **18** vorgesehen.

Der erste Kreislaufprozess liegt beim Durchlauf durch die Desorptionsstufe **6** in flüssiger Phase vor, während der zweite Kreislaufprozess zwar flüssig in die Desorptionsstufe eingeleitet wird, aber aus dieser nur mit dem gasförmigen Bestandteil über den Ablauf **15** zurück in den zweiten Prozesskreislauf zurückgeführt wird.

Ferner ist im ersten Kreislaufprozess ein Auslass **19** für das erste Gemisch aus Wasser und Methanol zwischen der Desorptionsstufe und dem ersten Wärmetauscher vorgesehen. Dies ist vorzugsweise der einzige Auslass für das sich in der Vorrichtung bildende Methanol aus den Kreislaufprozessen. Der hierüber abgezweigte Anteil am ersten Gemisch aus Wasser und Methanol wird vorzugsweise einer Destillationsapparatur **20** für die Trennung von Methanol und Wasser zugeführt.

Der **zweite Kreislaufprozess 2** mit einer zweiten umlaufenden Leitung **21** für ein umlaufendes zweites Gemisch aus Methanol, Wasser, Kohlendioxid und Wasserstoff durchläuft die vorgenannte Desorptionsstufe **6** für Kohlendioxid mit dem Zulauf für Wasserstoff **16,** gefolgt von einer ersten Flüssigkeits-Gas-Phasentrennstufe **22** mit einem Rücklauf für flüssige Phasen **23** des Methanols und des Wassers zu der Desorptionsstufe als Teil der zweiten umlaufenden Leitung sowie dem vorgenannten Gasauslass **15** und einem Einlass **14** aus der bzw. in die Desorptionsstufe.

Ferner weist der dargestellte zweite Kreislaufprozess zwischen Gasauslass **14** und Flüssigkeits-Gas-Phasentrennstufe **22** einen vorzugsweise vorgesehenen Kondensator **24** auf, in dem die gasförmig vorliegenden Bestandteile an Wasser und Methanol zumindest teilweise (weiter) verflüssigt werden, während das Kohlendioxid und der Wasserstoff in der gasförmigen Modifikation verbleiben und über einen Gasauslass **27** der ersten Flüssigkeits-Gas-Phasentrennstufe **22** in den nachfolgend beschrieben dritten Kreislaufprozess **3** übergeleitet werden.

Die erste Flüssigkeits-Gas-Phasentrennstufe **22** und der Kondensator **24** sind folglich nicht nur in den zweiten Kreislaufprozess **2,** sondern auch in den nachfolgend beschriebenen dritten Kreislaufprozess **3** eingebunden.

Der **dritte Kreislaufprozess 3** mit einer dritten umlaufenden Leitung **25** für ein umlaufendes drittes Gemisch aus Kohlendioxid und Wasserstoff umfasst einen Methanolsynthesereaktor **26** mit einer Kühlung **28,** die vorgenannte erste Flüssigkeits-Gas-Phasentrennstufe **22,** wobei der Gasauslass **27** in die dritte umlaufende Leitung **25** ausmündet, und die dritte umlaufende Leitung, die in einen Einlass **29** in die Flüssigkeits-Gas-Phasentrennstufe **22** einmündet. Die dritte umlaufende Leitung mündet - wie in **Fig.1** dargestellt - dabei vorzugsweise in die zweite umlaufende Leitung **21** zwischen Gasauslass **15** und dem optionalen Kondensator **27** oder dem Einlass **29** in die zweite umlaufende Leitung. Damit führt die dritte umlaufende Leitung über die zweite umlaufende Leitung in die Flüssigkeits-Gas-Phasentrennstufe **22.**

Ferner durchläuft der dritte Kreislaufprozess zwischen dem Methanolsynthesereaktor **26** und der ersten Flüssigkeits-Gas-Phasentrennstufe **22** einen zweiten Wärmetauscher **30,** wobei sich die dritte umlaufende Leitung in dem zweiten Wärmetauscher kreuzt. Ferner befindet sich zwischen dem Methanolsynthesereaktor **26** und dem zweiten Wärmetauscher **30** eine Fluidweiche **31** als Fluidauslass **38** in der dritten umlaufenden Leitung. Ferner ist ein Gebläse **32** im gasführenden Abschnitt der dritten umlaufenden Leitung zwischen dem zweiten Wärmetauscher **30** und dem Einlass **29** in die erste Flüssigkeits-Gas-Phasentrennstufe **22,** vorzugsweise dem Kondensator **24,** angeordnet.

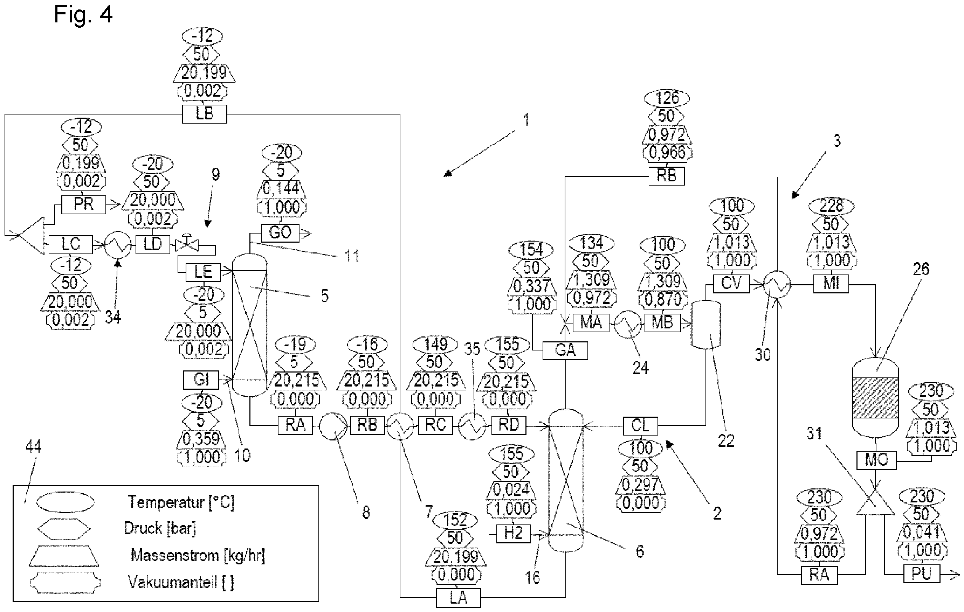

Die vorgenannte Vorrichtung ist in vorteilhafter Weise in nur zwei Druckniveaus betreibbar, wobei nur die Absorptionsstufe **5** im ersten Kreislaufprozess prinzipbedingt mit einem vorliegenden Rauchgas- oder Prozessgasdruck betrieben werden muss und vorzugsweiseweise für einen Bereich von 1 bis 10 bar ausgelegt ist. Alle Leitungsbereiche der drei Kreislaufprozesse der beidseitig in der ersten umlaufenden Leitung zur Absorptionsstufe angeordneten Umwälzpumpe **8** und Expansionsdrossel **9** sind für einen wesentlich höheren, an die Erfordernisse der Prozessbedingungen der Methanolsynthese aus CO₂ angepassten Druck ausgelegt. Jener liegt vorzugsweise einheitlich in einem Bereich von 40 bis 50 bar. Die Absorptionsstufe **5** wird dabei vorzugsweise in einem Temperaturbereich von -40°C bis 30°C, weiter bevorzugt von -30°C bis 10°C, die Desorptionsstufe **6** vorzugsweise in einem Temperaturbereich von -80°C bis 210°C, weiter bevorzugt von 100°C bis 180°C, weiter bevorzugt zwischen 140°C und 170°C, und der Methanolsynthesereaktor **26** vorzugsweise in einem Temperaturbereich von 180°C bis 300°C, weiter bevorzugt von 210°C bis 260°C betrieben. Die in **Fig.4** schematisch in einem Prozessfließbild des Verfahrens nach **Fig.1** dargestellten Zustandsgrößen liegen in diesen vorgenannten Intervallen.

Für die Temperatureinhaltung und -kontrolle der Temperatur in der Absorptionsstufe **5** und der Desorptionsstufe **6** sind vorzugsweise in der ersten umlaufenden Leitung **4** vor der Desorptionsstufe und/oder vor der Absorptionsstufe jeweils eine Temperierungsvorrichtung **34** bzw. **35** vorgeschaltet. Die Temperierungsvorrichtungen beeinflussen insbesondere die Eintrittstemperaturen des umlaufenden ersten Gemisches aus Wasser und Methanol in die Absorptionsstufe und Desorptionsstufe im ersten Kreislaufprozess und damit die vorgenannten Temperaturen in der Absorptionsstufe und der Desorptionsstufe. Die Temperierungsvorrichtung **34** vor der Absorptionsstufe ist vorzugsweise ein kombiniertes Kühl- und Heizelement oder ein Kühlelement. Die Temperierungsvorrichtung **35** vor der Desorptionsstufe ist vorzugsweise ein kombiniertes Kühl- und Heizelement oder ein Heizelement.

**Fig.2** zeigt schematisch das in **Fig.1** dargestellte Ausführungsbeispiel, jedoch ergänzt durch optionale zusätzliche Temperierungsmittel für das Verbrennungsabgas (oder das Prozessgas) sowie einen ebenso optionalen zusätzlichen Flüssigphasenrücklauf zu der Desorptionsstufe. Die nicht mit Bezugszeichen versehenen Komponenten entsprechen, sofern nicht anders angegeben, grundsätzlich den in **Fig.1** mit Bezugszeichen dargestellten Komponenten.

Die vorgenannten Temperierungsmittel umfassen insbesondere einen dritten Wärmetauscher **33.** In diesem sind einerseits das kohlendioxidhaltige Verbrennungsgas aus einem Prozess **36** vor dem Einlass **10** in die Absorptionsstufe, andererseits ein aus dem Auslass **11** der Absorptionsstufe herausgeleitetes und einer nachgeschalteten Rauchgasreinigung **37** zuführbares Verbrennungsgas in je einer Passagenfraktion durchleitbar. Es wird angestrebt, die Verbrennungsgase vor Eintritt in die Absorptionsstufe auf die Prozesstemperatur in der Absorptionsstufe zu temperieren, d.h. auf den vorgenannten Temperaturbereich von - 40°C bis 30°C, weiter bevorzugt von -30°C bis 10°C.

Der in **Fig.2** ebenfalls dargestellte zusätzliche Flüssigphasenrücklauf greift den an der Fluidweiche **31** aus dem dritten Kreislaufprozess **3** aus der dritten umlaufenden Leitung **25** zwischen dem Methanolsynthesereaktor **26** und dem zweiten Wärmetauscher **30** abzweigenden Fluidauslass **38** auf (vgl. **Fig.1****).** Der Fluidauslass mündet in einen Flüssigphasenrücklauf **39** zu der Desorptionsstufe **6** aus. Weiter bevorzugt weist der Flüssigphasenrücklauf selbst eine Förderpumpe **40** und/oder eine zweite Flüssigkeits-Gas-Phasentrennstufe **41** mit einem aus dieser abzweigenden Gasauslass **42** zur Abtrennung von Inertgasanteilen auf, sodass grundsätzlich nur die abgezweigten flüssigen Bestandteile in die Desoptionsstufe, bevorzugt in den oberen Bereich der Desorptionsstufe zurückgeführt werden. Vor der zweiten Flüssigkeits-Gas-Phasentrennstufe **41** ist im Flüssigphasenrücklauf vorzugsweise ein zweiter Kondensator **43** vorgeschaltet, der das Fluid im Flüssigphasenrücklauf auf eine Temperatur, vorzugsweise im in der Desorptionsstufe vorliegenden Temperaturbereich von -80°C bis 210°C, weiter bevorzugt von 100°C bis 180°C, weiter bevorzugt zwischen 140°C und 170°C einstellt und damit in vorteilhafter Weise eine exaktere stoffliche Trennung der flüssigen Phasen Wasser und Methanol von den gasförmigen Phasen Kohlenstoffdioxid und Wasserstoff ermöglicht.

**Fig.1** **und** **2** repräsentieren Ausgestaltungen, bei denen die Umwälzpumpe **8** zwischen der Absorptionsstufe **5** und dem ersten Wärmetauscher **7** angeordnet ist.

**Fig.3** repräsentiert eine weitere bevorzugte Ausgestaltung der Vorrichtung, die sich dadurch auszeichnet, dass der der erste Wärmetauscher **7** durch zwei (oder mehrere) in Reihe geschaltete Wärmetauschelemente **7a** und **7b** gebildet wird, wobei die Kanäle, d.h. die beiden Abschnitte der ersten umlaufenden Leitungen, für zwei Fluidfraktionen die beiden Wärmetauschelemente in gegenläufiger Reihenfolge durchdringen. Die Leitungsabschnitte zwischen den beiden Wärmetauschelementen dienen optional der Aufnahme der Umwälzpumpe **8** und/oder der Expansionsdrossel **9.**

Die in allen **Figuren** dargestellte Anordnung der Expansionsdrossel **9** zwischen Absorptionsstufe und erstem Wärmetauscher **7** ist bevorzugt, da damit die Druckabsenkung und damit Temperaturabsenkung in vorteilhafter Weise nach der Wärmeübertragung im ersten Wärmetauscher stattfindet.

**Fig.3** zeigt eine bevorzugte Anordnung der Umwälzpumpe **8** zwischen der Absorptionsstufe **5** und der Desorptionsstufe **6** zwischen den beiden Wärmetauschelementen **7a** und **7b.** Diese Ausgestaltung hat den Vorteil, dass ein Teil der ersten Leitung vor der Umwälzpumpe auf einem niedrigerem Druck- und damit Temperaturniveau zumindest das erste Wärmetauschelement **7a** durchläuft und damit dort ein erhöhtes Temperaturgefälle und damit ein erhöhter Wärmeübergang realisierbar ist. Ferner lässt sich durch die Dimensionierung der beiden Wärmetauschelemente **7a** und **7b** das Temperaturniveau der zwischengelagerten Abschnitte der ersten umlaufenden Leitung exakt auf eine maximal mögliche Betriebstemperatur der Umwälzpumpe **8** und/oder der Expansionsdrossel **9** einstellen.

Eine nicht weiter dargestellte, aber thermodynamisch besonders günstige Ausgestaltung sieht eine Anordnung der Umwälzpumpe **8** zwischen dem ersten Wärmetauscher **7** und der Desorptionsstufe **6** vor. Die erste umlaufende Leitung durchdringt in Richtung der Absorptionsstufe den ersten Wärmetauscher vor der Expansionsdrossel, d.h. noch mit dem erhöhten Druck, d.h. erhöhter Temperatur der Desorptionsstufe. Dagegen erreicht die erste umlaufende Leitung in Richtung der Desorptionsstufe zunächst mit dem relativ niedrigen Druck und der relativ niedrigen Temperatur der Absorptionsstufe den ersten Wärmetauscher, bevor in der nachgeschalteten Umwälzpumpe Druck und Temperatur weiter erhöht werden. Im ersten Wärmetauscher wird mit dieser Ausgestaltung ein erhöhtes Temperaturgefälle realisiert. Allerdings begrenzt die maximale Betriebstemperatur der Umwälzpumpe die Einsetzbarkeit, weswegen die in **Fig.3** dargestellte Ausgestaltung mit einer Anordnung der Umwälzpumpe zwischen den beiden Wärmetauschelementen **7a** und **7b** zu bevorzugen ist.

Der erste, zweite und dritte Wärmetauscher sowie die genannten Wärmetauschelemente sind jeweils Oberflächenwärmtauscher, mit jeweils zwei Fluidkanälen oder Fluidkanalfraktionen, weiter bevorzugt Gegen- oder Kreuzstromwärmetauscher. Eine unterschiedliche Beladung der Fluidfraktionen in den Wärmetauschern schließt einen Einsatz von Mischwärmetauschern aufgrund der dabei naturgemäß vorgesehenen stofflichen Vermischungen aus.

Die in **Fig.2** **und** **3** dargestellten Ausgestaltungen sind miteinander kombinierbar.

Für die Durchführung des **Verfahrens** zur Herstellung von Methanol aus Kohlendioxid, erfolgt zunächst die Bereitstellung einer Vorrichtung nach einer der vorgenannten Ausführungen. Über den Einlass **10** wird ein kohlendioxidhaltiges Gas durch die Absorptionsstufe **5** hindurch und durch den Auslass **11** wieder ausgeleitet, wobei das Kohlendioxid bevorzugt bei einem der für die Absorptionsstufe genannten Drücke und Temperaturen von einem flüssigen ersten Gemisch aus Wasser und Methanol in den ersten Kreislaufprozess **1** aufgenommen wird. Es folgt eine Komprimierung und Temperierung des ersten Gemisches mit dem Kohlendioxid in der Umwälzpumpe **8** bzw. im ersten Wärmetauscher **7** des ersten Kreislaufprozesses **1** auf einen bzw. eine für die Desorptionsstufe vorzugsweise genannten Druck bzw. Temperatur. Die Anordnung der Umwälzpumpe und des ersten Wärmetauschers wurde zuvor diskutiert.

Es folgt eine Einleitung des komprimierten und temperierten ersten Gemisches mit dem Kohlendioxid in die Desorptionsstufe **6,** wobei das Kohlendioxid aus dem ersten Gemisch desorbiert und dem ersten Kreislaufprozess entnommen und unter Hinzufügung von Wasserstoff von einem umlaufenden zweiten Gemisch aus Methanol, Wasser, Kohlendioxid und Wasserstoff im zweiten Kreislaufprozess **2** in einem für die Desorptionsstufe vorgeschlagenen Bereich für den Druck und die Temperatur aufgenommen wird. Das Kohlenstoffdioxid CO₂ wird dabei aufgrund der erhöhten Temperatur und eines über den Zulauf **16** am Boden der Desorptionsstufe injizierten Wasserstoffstroms aus dem Methanol/Wasser-Gemisch desorbiert. Der Wasserstoffstrom hat dabei zwei Funktionen. Einerseits wird er als Spülgas genutzt und erhöht damit die CO₂-Desorption im bevorzugt säulenförmigen Volumen in der Desorptionsstufe. Andererseits dient er dem Eintrag von Wasserstoff als Ausgangsstoff für eine Methanolsynthese im Methanolsyntesereaktor **26,** in der das Kohlenstoffdioxid und der Wasserstoff zu Methanol und Wasser umgesetzt werden. Hierzu wird Kohlenstoffdioxid und der Wasserstoff über den Gasauslass **15** aus der Desorptionsstufe **6** in den zweiten Kreislaufprozess **2** ausgeleitet. Von dort werden die Gase über die erste Flüssigkeits-Gas-Phasentrennstufe **22** als gasförmige drittes Gemisch über den Gasauslass **27** selektiv in den dritten Kreislaufprozess **3** weitergeleitet. Es folgt im dritten Kreislaufprozess nach Durchlauf durch den zweiten Wärmetauscher **30** aufgewärmt eine Überführung zum Methanolsyntesereaktor **26.** Dort findet eine möglichst vollständige, zumindest aber eine partielle Umsetzung des dritten Gemisches zu Methanol und Wasser statt.

Das gasförmige dritte Gemisch aus Wasserstoff und Kohlenstoffdioxid (das ggf. noch Restbestände an Methanol und Wasser enthält) wird dabei vorzugswiese oben auf das Innenvolumen des Methanolsyntesereaktors in Richtung des Ausgangs des Methanolsynthesereaktors auf das bereits gebildete Wasser und Methanol aufgespritzt, wobei es zu einer kochenden Einmischung und einer beschleunigten Umsetzung zu Wasser und Methanol kommt.

Wasserstoff, H₂, und Kohlendioxid, CO₂, reagieren im Methanolsynthesereaktor unter Bildung von Methanol und Wasser. Die Reaktorausgangstemperatur wird durch einen Gegenstromfluss einer Art thermischer Flüssigkeit (z. B. kochendes Wasser oder eine andere Flüssigkeit) gesteuert. Die Steuerung der Temperatur am Ausgang des Reaktors ist aus thermodynamischen Gründen wichtig, um die Ausbeute an Methanol zu maximieren.

Das den Methanolsyntesereaktor **26** über einen unten im Innenvolumen angeordneten Auslass verlassende Methanol und Wasser sowie der nicht umgesetzte Teil des dritten Gemisches wird dann zur Temperierung durch den zweiten Wärmetauscher **30** geleitet, wo er einen Wärmeüberschuss an den im dritten Kreislaufprozess **3** zum Methanolsyntesereaktor strebenden Massenstrom des dritten Gemisches abgibt und in die erste Flüssigkeits-Gas-Phasentrennstufe **22** zurückgeleitet wird. Dort erfolgt eine laufende Abtrennung der flüssigen Bestandteile des Gemisches, des Methanols und des Wassers und Rückführung dieser über den zweiten Kreislaufprozess **2** in die Desorptionsstufe **6** sowie eine kontinuierliche Abzweigung eines Gemisches aus Wasser und Methanol aus der Desorptionsstufe **6.** Der verbleibende, nicht umgesetzte Teil des dritten Gemisches wird dagegen als Gas in der ersten Flüssigkeits-Gas-Phasentrennstufe **22** abgesondert in den dritten Kreislaufprozess und in diesem zum Methanolsynthesereaktor zurückgeführt.

Eine bevorzugte Ausgestaltung des Verfahrens umfasst zudem ein Durchleiten des vorgenannten dritten Gemisches (H₂ und CO₂) sowie des Methanols und des Wassers durch eine Fluidweiche **31** im dritten Kreislaufprozess **3** zwischen dem Methanolsynthesereaktor **26** und dem zweiten Wärmetauscher **30,** wobei ein Teil des dritten Gemisches, des Methanols und des Wassers in einen Fluidauslass **38** umgelenkt wird. Diese partielle Abtrennung von Fluidbestandteilen dient vorzugsweise der laufenden Abtrennung auch von unerwünschten gasförmigen Reaktionsprodukten und Verunreinigungen aus dem dritten Kreislaufprozess und damit auch aus der Methanolsynthese, die ansonsten als Gase in der ersten Flüssigkeits-Gas-Phasentrennstufe **22** laufend im dritten Kreislaufprozess zurückgeleitet werden, damit den Prozesskreislauf nicht verlassen könnten und so in diesem zu einer unerwünschten Anreicherung führen würden.

Vorzugsweise wird der in der Fluidweiche abgetrennte Teil des dritten Gemisches, des Methanols und des Wassers aus dem Fluidauslass **38** über einen Flüssigphasenrücklauf **39** zu der Desorptionsstufe **6,** d.h. in den Wirkbereich der ersten beiden Prozesskreisläufe rückgeführt. Weiter bevorzugt wird dabei aber der Teil des dritten Gemisches, des Methanols und des Wassers im Flüssigphasenrücklauf **39** mit einer Förderpumpe **40** komprimiert und/oder aber insbesondere in der zweiten Flüssigkeits-Gas-Phasentrennstufe **41** von unerwünschten Gasanteilen befreit.

**Fig.4** zeigt schematisch ein Prozessfließbild des Verfahrens in einer Vorrichtung gemäß der in **Fig.1** dargestellten Ausführungsform. Die Legende **44** umfasst die Formsymbole für mehrere Zustandsdaten im Diagramm: von oben nach unten für die Temperatur in [°C], den Druck in [bar], den Massenstrom in [kg/h] sowie den Vakuumanteil in [-]. Die Formsymbole finden sich im Diagramm in allen drei Kreislaufprozessen **1 bis 3** an verschiedenen Stellen mit den dort vorliegenden Zustandsdaten wieder. Wesentliche Komponenten der Vorrichtung sind mit den bereits aus **Fig.1** bekannten Bezugszeichen versehen.

### Bezugszeichenliste:

- 1: erster Kreislaufprozess
- 2: zweiter Kreislaufprozess
- 3: dritter Kreislaufprozess
- 4: erste umlaufende Leitung
- 5: Absorptionsstufe
- 6: Desorptionsstufe
- 7: erster Wärmetauscher
- 8: Umwälzpumpe
- 9: Expansionsdrossel
- 10: Einlass für ein kohlendioxidhaltiges Gas in die Absorptionsstufe
- 11: Auslass für ein kohlendioxidhaltiges Gas aus der Absorptionsstufe
- 12: Zulauf des ersten Kreislaufprozesses in die Desorptionsstufe
- 13: Ablauf des ersten Kreislaufprozesses aus der Desorptionsstufe
- 14: Einlass für flüssige Bestandteile des zweiten Kreislaufprozesses in die Desorptionsstufe
- 15: Gasauslass aus der Desorptionsstufe
- 16: Zulauf für Wasserstoff in die Desorptionsstufe
- 17: Wasserstoffquelle
- 18: Temperierungsmittel für Wasserstoff
- 19: Auslass für ein Gemisch aus Wasser und Methanol aus dem ersten Kreislaufprozess
- 20: Destillationsapparatur
- 21: zweite umlaufende Leitung
- 22: erste Flüssigkeits-Gas-Phasentrennstufe
- 23: Rücklauf für flüssige Phasen zu der Desorptionsstufe
- 24: Kondensator
- 25: dritte umlaufende Leitung
- 26: Methanolsynthesereaktor
- 27: Gasauslass aus der ersten Flüssigkeits-Gas-Phasentrennstufe
- 28: Kühlung
- 29: Einlass in die Flüssigkeits-Gas-Phasentrennstufe
- 30: zweite Wärmetauscher
- 31: Fluidweiche
- 32: Gebläse
- 33: dritte Wärmetauscher
- 34: Temperierungsvorrichtung vor Absorptionsstufe
- 35: Temperierungsvorrichtung vor Desorptionsstufe
- 36: Prozess
- 37: Rauchgasreinigung
- 38: Fluidauslass
- 39: Flüssigphasenrücklauf
- 40: Förderpumpe
- 41: zweite Flüssigkeits-Gas-Phasentrennstufe
- 42: Gasauslass aus der zweiten Flüssigkeits-Gas-Phasentrennstufe
- 43: Kondensator im Flüssigphasenrücklauf
- 44: Legende des Prozess-Fließbildes

## Patentansprüche

1. Vorrichtung zur Herstellung von Methanol aus Kohlendioxid, umfassend
a) einen ersten Kreislaufprozess **(1)** mit einer ersten umlaufenden Leitung **(4)** für ein umlaufendes erstes Gemisch aus Wasser und Methanol, umfassend
i) eine Absorptionsstufe **(5)** für Kohlendioxid mit einem Durchlass für ein kohlendioxidhaltiges Gas,
ii) eine Desorptionsstufe **(6)** für Kohlendioxid mit einem Zulauf **(16)** für Wasserstoff,
iii) einem ersten Wärmetauscher **(7)** zwischen der Absorptionsstufe und der Desorptionsstufe, wobei sich die umlaufende Leitung im Wärmetauscher kreuzt,
iv) einen Auslass **(19)** für das erste Gemisch aus Wasser und Methanol zwischen der Desorptionsstufe und dem ersten Wärmetauscher,
v) eine Umwälzpumpe **(8)** zwischen der Absorptionsstufe und der Desorptionsstufe sowie
vi) eine Expansionsdrossel **(9)** zwischen dem ersten Wärmetauscher und der Absorptionsstufe,
b) einen zweiten Kreislaufprozess **(2)** mit einer zweiten umlaufenden Leitung **(21)** für ein umlaufendes zweites Gemisch aus Methanol, Wasser, Kohlendioxid und Wasserstoff, umfassend
i) die Desorptionsstufe **(6)** für Kohlendioxid mit dem Zulauf **(19)** für Wasserstoff,
ii) eine erste Flüssigkeits-Gas-Phasentrennstufe **(22)** mit einem Rücklauf **(23)** für flüssige Phasen des Methanols und des Wassers zu der Desorptionsstufe als Teil der zweiten umlaufenden Leitung sowie
iii) einen Gasauslass **(15)** aus der Desorptionsstufe und einen Einlass **(14)** für die flüssige Phasen des Methanols und des Wassers in die Desorptionsstufe sowie
c) einen dritten Kreislaufprozess **(3)** mit einer dritten umlaufenden Leitung **(25)** für ein umlaufendes drittes Gemisch aus Kohlendioxid und Wasserstoff, umfassend
i) einen Methanolsynthesereaktor **(26)** mit einer Kühlung **(28),**
ii) die erste Flüssigkeits-Gas-Phasentrennstufe **(22),** wobei der Gasauslass **(27)** in die dritte umlaufende Leitung ausmündet und die dritte umlaufende Leitung in den Einlass **(29)** einmündet,
iii) einen zweiten Wärmetauscher **(30)** zwischen dem Methanolsynthesereaktor und der ersten Flüssigkeits-Gas-Phasentrennstufe, wobei sich die dritte umlaufende Leitung in dem zweiten Wärmetauscher kreuzt,
iv) einen Gasauslass **(31)** in der dritten umlaufenden Leitung zwischen dem Methanolsynthesereaktor und dem zweiten Wärmetauscher,
v) ein Gebläse **(32)** zwischen dem zweiten Wärmetauscher und dem Einlass die erste Flüssigkeits-Gas-Phasentrennstufe.

2. Vorrichtung nach Anspruch 1, **dadurch gekennzeichnet, dass** in der zweiten und/oder dritten umlaufenden Leitung **(21, 25)** zwischen der Desorptionsstufe **(6)** beziehungsweise dem zweiten Wärmetauscher **(30)** einerseits und der erste Flüssigkeits-Gas-Phasentrennstufe **(22)** andererseits ein Kondensator **(24)** vorgesehen ist.

3. Vorrichtung nach Anspruch 1 oder 2, **dadurch gekennzeichnet, dass** in der dritten umlaufenden Leitung **(25)** zwischen dem Methanolsynthesereaktor **(26)** und dem zweiten Wärmetauscher **(30)** eine Fluidweiche **(31)** vorgesehen ist, wobei ein Fluidrücklauf als Teil der dritten umlaufenden Leitung zu dem zweiten Wärmetauscher sowie ein Fluidauslass **(38)** vorgesehen ist.

4. Vorrichtung nach Anspruch 3, **dadurch gekennzeichnet, dass** der Fluidauslass **(38)** in einen Flüssigphasenrücklauf **(39)** zu der Desorptionsstufe **(6)** ausmündet.

5. Vorrichtung nach Anspruch 4, **dadurch gekennzeichnet, dass** der Flüssigphasenrücklauf **(39)** eine Förderpumpe **(40)** und/oder eine zweite Flüssigkeits-Gas-Phasentrennstufe **(41)** mit einem Gasauslass **(42)** zur Abtrennung von Inertgasanteilen aufweist.

6. Vorrichtung nach einem der vorgenannten Ansprüche, **dadurch gekennzeichnet, dass** die Absorptionsstufe **(5)** ein Nasswäscher ist.

7. Vorrichtung nach einem der vorgenannten Ansprüche, **dadurch gekennzeichnet, dass** die Desorptionsstufe **(6)** ein Volumen umfasst, das als Mischkammer in den ersten und zweiten Kreislaufprozess **(4, 21)** integriert ist.

8. Vorrichtung nach einem der vorgenannten Ansprüche, **dadurch gekennzeichnet, dass** der erste Wärmetauscher **(7)** durch zwei in Reihe geschalte Wärmetauschelemente **(7a, 7b)** gebildet wird, wobei die Kanäle für zwei Fluidfraktionen die beiden Wärmetauschelemente in gegenläufiger Reihenfolge durchdringen.

9. Vorrichtung nach Anspruch 8, **dadurch gekennzeichnet, dass** die Umwälzpumpe **(8)** zwischen der Absorptionsstufe **(5)** und der Desorptionsstufe **(6)** zwischen den beiden Wärmetauschelementen **(7a, 7b)** angeordnet ist.

10. Vorrichtung nach einem der Ansprüche 1 bis 8, **dadurch gekennzeichnet, dass** die Umwälzpumpe **(8)** zwischen der Absorptionsstufe **(5)** und dem ersten Wärmetauscher **(7)** angeordnet ist.

11. Vorrichtung nach einem der Ansprüche 1 bis 8, **dadurch gekennzeichnet, dass** die Umwälzpumpe **(8)** zwischen dem ersten Wärmetauscher **(7)** und der Desorptionsstufe **(6)** angeordnet ist.

12. Vorrichtung nach einem der vorgenannten Ansprüche, **dadurch gekennzeichnet, dass** in der ersten umlaufenden Leitung **(4)** vor der Desorptionsstufe **(6)** und/oder vor der Absorptionsstufe **(5)** jeweils eine Temperierungsvorrichtung **(34, 35)** vorgeschaltet ist.

13. Verfahren zur Herstellung von Methanol aus Kohlendioxid, umfassend folgende Verfahrensschritte:
a) Bereitstellung einer Vorrichtung nach einem der vorgenannten Ansprüche 1 bis 12,
b) Durchleitung eines kohlendioxidhaltigen Gases durch die Absorptionsstufe **(5),** wobei das Kohlendioxid bei einem Druck zwischen 1 und 10 bar und einer Temperatur zwischen -30 und 10°C von einem flüssigen ersten Gemisch aus Wasser und Methanol in den ersten Kreislaufprozess **(1)** aufgenommen wird,
c) Komprimierung und Temperierung des ersten Gemisches mit dem Kohlendioxid in der Umwälzpumpe **(8)** bzw. im ersten Wärmetauscher **(7)** des ersten Kreislaufprozesses **(1)** auf einen Druck zwischen 40 und 50 bar und einer Temperatur zwischen 100 und 180°C,
d) Einleitung des komprimierten und temperierten ersten Gemisches mit dem Kohlendioxid in die Desorptionsstufe **(6),** wobei das Kohlendioxid aus dem ersten Gemisch desorbiert und dem ersten Kreislaufprozess entnommen wird und unter Hinzufügung von Wasserstoff von einem umlaufenden zweiten Gemisch aus Methanol, Wasser, Kohlendioxid und Wasserstoff im zweiten Kreislaufprozess **(2)** bei einem Druck zwischen 40 und 50 bar und einer Temperatur zwischen 100 und 180°C aufgenommen wird,
e) Weiterleitung des Kohlendioxids im zweiten Kreislaufprozess **(2)** in die erste Flüssigkeits-Gas-Phasentrennstufe **(22),** in der ein Gasgemisch aus Kohlendioxid und Wasserstoff selektiv als drittes Gemisch vom zweiten in den dritten Kreislaufprozess **(3)** umgeleitet wird,
f) Aufwärmung des Gasgemisches im zweiten Wärmetauscher **(30)** im dritten Kreislaufprozess auf eine Temperatur zwischen 210 und 260°C,
g) Einleitung des temperierten Gasgemisches in den Methanolsynthesereaktor **(26)** und partielle Umsetzung des dritten Gemisches zu Methanol und Wasser,
h) Zurückleitung des dritten Gemisches, des Methanols und des Wassers durch den zweiten Wärmetauscher **(30)** hindurch in die erste Flüssigkeits-Gas-Phasentrennstufe **(22),**
i) Abtrennung der flüssigen Bestandteile des Gemisches, des Methanols und des Wassers in der ersten Flüssigkeits-Gas-Phasentrennstufe **(22)** und Rückführung dieser über den zweiten Kreislaufprozess **(2)** in die Desorptionsstufe **(6)** sowie
j) Kontinuierliche Abzweigung eines Gemisches aus Wasser und Methanol aus der Desorptionsstufe **(6).**

14. Verfahren nach Anspruch 13, umfassend ein Durchleiten des dritten Gemisches, des Methanols und des Wassers durch eine Fluidweiche **(31)** im dritten Kreislaufprozess **(3)** zwischen dem Methanolsynthesereaktor **(26)** und dem zweiten Wärmetauscher **(30),** wobei ein Teil des dritten Gemisches, des Methanols und des Wassers in einen Fluidauslass **(38)** umgelenkt wird.

15. Verfahren nach Anspruch 14, **dadurch gekennzeichnet, dass** ein Teil des dritten Gemisches, des Methanols und des Wassers aus dem Fluidauslass **(38)** über einen Flüssigphasenrücklauf **(39)** zu der Desorptionsstufe **(6)** zurückgeführt wird.

16. Verfahren nach Anspruch 15, **dadurch gekennzeichnet, dass** der Teil des dritten Gemisches, des Methanols und des Wassers im Flüssigphasenrücklauf **(39)** mit einer Förderpumpe **(40)** komprimiert und/oder in einer zweite Flüssigkeits-Gas-Phasentrennstufe **(41)** von Gasanteilen befreit wird.

17. Verfahren nach einem der Ansprüche 13 bis 16, **dadurch gekennzeichnet, dass** in der ersten umlaufenden Leitung **(4)** vor der Desorptionsstufe **(6)** und/oder vor der Absorptionsstufe **(5)** jeweils eine Temperaturkontrolle oder Temperierung **(34, 35)** im ersten Kreislaufprozess **(1)** erfolgt.

## Claims

1. Device for producing methanol from carbon dioxide, comprising
a) a first circulation process (1) with a first circumferential line (4) for a circulating first mixture of water and methanol, comprising
i) an absorption stage (5) for carbon dioxide with a through passage for a gas containing carbon dioxide,
ii) a desorption stage (6) for carbon dioxide with an inlet (16) for hydrogen,
iii) a first heat exchanger (7) between the absorption stage and the desorption stage, wherein the circumferential line crosses in the heat exchanger,
iv) an outlet (19) for the first mixture of water and methanol between the desorption stage and the first heat exchanger,
v) a circulation pump (8) between the between the absorption stage and the desorption stage, and
vi) an expansion throttle (9) between the first heat exchanger and the absorption stage,
b) a second circulation process (2) with a second circumferential line (21) for a circulating second mixture of methanol, water, carbon dioxide, and hydrogen, comprising
i) the desorption stage (6) for carbon dioxide with the inlet (16) for hydrogen,
ii) a first liquid-gas phase separation stage (22) with a return (23) for liquid phases of the methanol and of the water to the desorption stage as a part of the second circumferential line, and
iii) a gas outlet (15) out of the desorption stage and an inlet (14) for the liquid phases of the methanol and of the water into the desorption stage, and
c) a third circulation process (3) with a third circumferential line (25) for a circulating third mixture of carbon dioxide and hydrogen, comprising
i) a methanol synthesis reactor (26) with a cooling device (28),
ii) the first liquid-gas phase separation stage (22), wherein the gas outlet (27) opens out into the third circumferential line and the third circumferential line opens into the inlet (29),
iii) a second heat exchanger (30) between the methanol synthesis reactor and the first liquid-gas phase separation stage, wherein the third circumferential line crosses in the second heat exchanger,
iv) a gas outlet (31) in the third circumferential line between the methanol synthesis reactor and the second heat exchanger,
v) a fan (32) between the second heat exchanger and the inlet into the first liquid-gas phase separation stage.

2. Device according to claim 1, **characterised in that** a condenser (24) is provided in the second and/or third circumferential line (21, 25) between the desorption stage (6) or the second heat exchanger (30) respectively on the one hand, and the first liquid-gas phase separation stage (22) on the other hand.

3. Device according to claim 1 or 2, **characterised in that** a fluid divider (31) is provided in the third circumferential line (25) between the methanol synthesis reactor (26) and the second heat exchanger (30), wherein a fluid return is provided as a part of the third circumferential line to the second heat exchanger, as well as a fluid outlet (38).

4. Device according to claim 3, **characterised in that** the fluid outlet (38) opens out into a liquid phase return (39) to the desorption stage (6).

5. Device according to claim 4, **characterised in that** the liquid phase return (39) comprises a delivery pump (40) and/or a second liquid-gas separation stage (41) with a gas outlet (42) for the separation of inter gas portions.

6. Device according to any one of the preceding claims, **characterised in that** the absorption stage (5) is a wet washer.

7. Device according to any one of the preceding claims, **characterised in that** the desorption stage (6) comprises a volume which is integrated as a mixing chamber into the first and second circulation process (4, 21).

8. Device according to any one of the preceding claims, **characterised in that** the first heat exchanger (7) is formed by two heat exchange elements (7a, 7b) connected in series, wherein the channels for two fluid fractions penetrate the two heat exchange elements in a counter-sequence.

9. Device according to claim 8, **characterised in that** the circulating pump (8) between the adsorption stage (5) and the desorption stage (6) is arranged between the two heat exchange elements (7a, 7b).

10. Device according to any one of claims 1 to 8, **characterised in that** the circulation pump (8) is arranged between the adsorption stage (5) and the first heat exchanger (7).

11. Device according to any one of claims 1 to 8, **characterised in that** the circulation pump (8) is arranged between the first heat exchanger (7) and the desorption stage (6).

12. Device according to any one of the preceding claims, **characterised in that** in each case a temperature control device (34, 35) is arranged in the first circumferential line (4) upstream of the desorption stage (6) and/or upstream of the absorption stage (5).

13. Method for producing methanol from carbon dioxide, comprising the following method steps:
a) provision of a device in accordance with any one of the preceding claims 1 to 12,
b) conveying of a gas containing carbon dioxide through the absorption stage (5), wherein the carbon dioxide is received into the first circulation process (1) at a pressure of between 1 and 10 bar and a temperature of between -30 and 10 °C from a liquid first mixture of water and methanol,
c) compressing and temperature-controlling of the first mixture with the carbon dioxide in the circulation pump (8) or in the first heat exchanger (7) of the first circulation process (1) to a pressure of between 40 and 50 bar and a temperature of between 100 and 180 °C,
d) Introduction of the compressed and temperature-controlled first mixture with the carbon dioxide into the desorption stage (6), wherein the carbon dioxide is desorbed from the first mixture, and removed from the first circulation process, and, with the addition of hydrogen, is taken up from a circulating second mixture of methanol, water, carbon dioxide and hydrogen in the second circulation process (2) at a pressure of between 40 and 50 bar and at a temperature of between 100 and 180 °C,
e) further conveying of the carbon dioxide in the second circulation process (2) into the first liquid-gas phase separation stage (22), in which a gas mixture of carbon dioxide and hydrogen is diverted selectively as a third mixture from the second circulation process into the third circulation process (3),
f) heating of the gas mixture in the second heat exchanger (30) in the third circulation process to a temperature of between 210 and 260 °C,
g) introduction of the temperature-controlled gas mixture into the methanol synthesis reactor (26), and partial conversion of the third mixture into methanol and water,
h) conveying of the third mixture of the methanol, and of the water through the second heat exchanger (30) into the first liquid-gas phase separation stage (22),
i) separation of the liquid constituent parts of the mixture, of the methanol, and of the water in the first liquid-gas phase separation stage (22) and conveying of these back via the second circulation process (2) into the desorption stage (6), and
j) continuous branching of a mixture of water and methanol out of the desorption stage (6).

14. Method according to claim 13, comprising a conveying of the third mixture of the methanol, and of the water through a fluid branch (31) in the third circulation process (3) between the methanol synthesis reactor (26) and the second heat exchanger (30), wherein a part of the third mixture of the methanol, and of the water is diverted into a fluid outlet (38).

15. Method according to claim 14, **characterised in that** a part of the third mixture of the methanol, and of the water is conveyed back out of the fluid outlet (38) via a liquid phase return (39) to the desorption stage (6).

16. Method according to claim 15, **characterised in that** the part of the third mixture of the methanol, and of the water is compressed in the liquid phase return (39) with a delivery pump (40) and/or is freed of gas portions in a second liquid-gas phase separation stage (41).

17. Method according to any one of claims 13 to 16, **characterised in that** in the first circumferential line (4) in each case temperature monitoring or temperature controlling (34, 35) takes place in the first circulation process (1) upstream of the desorption stage (6) and/or upstream of the absorption stage (5).

## Revendications

1. Dispositif de production de méthanol à partir de dioxyde de carbone comprenant :
a) une première boucle de procédé (1) avec une première conduite de circulation (4) pour un premier mélange d'eau et de méthanol en circulation comprenant :
i) un étage d'absorption (5) du dioxyde de carbone avec un passage d'un gaz contenant du dioxyde de carbone,
ii) un étage de désorption (6) du dioxyde de carbone avec une arrivée (16) d'hydrogène,
iii) un premier échangeur de chaleur (7) entre l'étage d'absorption et l'étage de désorption,
* la conduite de circulation traversant l'échangeur de chaleur,
iv) une sortie (19) pour un premier mélange d'eau et de méthanol entre l'étage de désorption et le premier échangeur de chaleur,
v) une pompe de circulation (8) entre l'étage d'absorption et l'étage de désorption, et
vi) un étranglement d'expansion (9) entre le premier échangeur de chaleur et l'étage d'absorption,
b) une seconde boucle de procédé (2) avec une seconde conduite de circulation (21) pour un second mélange en circulation composé de méthanol, d'eau, de dioxyde de carbone et d'hydrogène, comprenant :
i) un étage de désorption (6) du dioxyde de carbone avec l'arrivée d'hydrogène (16),
ii) un premier étage de séparation de phases liquide/gaz (22) avec un retour (23) pour les phases liquides du méthanol et de l'eau pour l'étage de désorption comme partie de la seconde conduite de circulation, et
iii) une sortie de gaz (15) de l'étage de désorption et une entrée (14) pour les phases liquides du méthanol et de l'eau dans l'étage de désorption ainsi que,
c) une troisième boucle de procédé (3) avec une troisième conduite de circulation (25) pour un troisième mélange en circulation de dioxyde de carbone et d'hydrogène comprenant :
i) un réacteur de synthèse de méthanol (26) avec un refroidissement (28),
ii) le premier étage de séparation de phases liquide/gaz (22),
* la sortie de gaz (27) débouchant dans la troisième conduite de circulation et cette troisième conduite de circulation débouchant dans l'entrée (29),
iii) un second échangeur de chaleur (30) entre le réacteur de synthèse de méthanol et le premier étage de séparation de phase liquide/gaz,
* la troisième conduite de circulation traversant le second échangeur de chaleur,
iv) une sortie de gaz (31) dans la troisième conduite de circulation entre le réacteur de synthèse de méthanol et le second échangeur de chaleur,
v) une soufflante (32) entre le second échangeur de chaleur et l'entrée du premier étage de séparation de phase liquide/gaz.

2. Dispositif selon la revendication 1,
**caractérisé par**
un condenseur (24) dans la seconde et/ou la troisième conduite de circulation (21, 25), entre d'une part entre l'étage de désorption (6) ou le second échangeur de chaleur (30) et d'autre part le premier étage séparateur de phase liquide/gaz (22).

3. Dispositif selon la revendication 1 ou 2,
**caractérisé par**
une dérivation de fluide (31) dans la troisième conduite de circulation (25) entre le réacteur de synthèse de méthanol (26) et le second échangeur de chaleur (30) avec un retour de fluide comme partie de la troisième conduite de circulation vers le second échangeur de chaleur et une sortie de fluide (38).

4. Dispositif selon la revendication 3,
**caractérisé en ce que**
la sortie de fluide (38) débouche dans un retour de phase liquide (39) vers l'étage de désorption (6).

5. Dispositif selon la revendication 4,
**caractérisé en ce que**
le retour de phase liquide (39) comporte une pompe (40) et/ou un second étage séparateur de phases liquide/gaz (41) avec une sortie de gaz (42) pour séparer les parties de gaz inerte.

6. Dispositif selon l'une des revendications précédentes,
**caractérisé en ce que**
l'étage d'absorption (5) est un laveur humide.

7. Dispositif selon l'une des revendications précédentes,
**caractérisé en ce que**
l'étage de désorption (6) a un volume qui est intégré comme chambre de mélange dans la première et dans la seconde boucle de procédé (4, 21).

8. Dispositif selon l'une des revendications précédentes,
**caractérisé en ce que**
le premier échangeur de chaleur (7) est formé par deux éléments d'échangeur de chaleur (7a, 7b) branchés en série,
* les canaux pour les deux fractions de fluide traversant à contre-courant les deux éléments échangeurs de chaleur.

9. Dispositif selon la revendication 8,
**caractérisé en ce que**
la pompe de circulation (8) est installée entre l'étage d'adsorption (5) et l'étage de désorption (6) entre les deux éléments d'échangeur de chaleur (7a, 7b).

10. Dispositif selon l'une des revendications 1 à 8,
**caractérisé par**
la pompe de circulation (8) entre l'étage d'adsorption (5) et le premier échangeur de chaleur (7).

11. Dispositif selon l'une des revendications 1 à 8,
**caractérisé en ce que**
la pompe de circulation (8) est installée entre le premier échangeur de chaleur (7) et l'étage de désorption (6).

12. Dispositif selon l'une des revendications précédentes,
**caractérisé par**
un dispositif de mise en température (34, 35) respectif en amont de l'étage de désorption (6) dans la première conduite de circulation (4) et/ou en amont de l'étage d'absorption (5).

13. Procédé de production de méthanol à partir de dioxyde de carbone comprenant les étapes suivantes consistant à :
a) fournir un dispositif selon l'une des revendications 1 à 12,
b) faire passer un gaz contenant du dioxyde de carbone par l'étage d'absorption (5),
* le dioxyde de carbone étant à une pression comprise entre 1 et 10 bars et à une température comprise entre -30 et 10°C dans un premier mélange liquide d'eau et de méthanol dans la première boucle de procédé (1),
c) comprimer et mettre en température le premier mélange avec le dioxyde de carbone par la pompe de circulation (8) ou dans le premier échangeur de chaleur (7) de la première boucle de procédé (1) à une pression comprise entre 40 et 50 bars et une température comprise entre 100 et 180°C,
d) introduire le premier mélange comprimé et mis en température avec le dioxyde de carbone dans l'étage de désorption (6),
* le dioxyde de carbone étant désorbé du premier mélange et prélevé de la première boucle de procédé et par adjonction d'hydrogène reçu par un second mélange en circulation de méthanol d'eau et de dioxyde de carbone et d'hydrogène dans une seconde boucle de procédé (2) à une pression comprise entre 40 et 50 bars et une température comprise entre 100 et 180°C,
e) transférer le dioxyde de carbone dans une seconde boucle de circuit (2) dans le premier étage de séparation de phases liquide/gaz (22) dans laquelle un mélange gazeux de dioxyde de carbone et d'hydrogène est transféré sélectivement comme troisième mélange de la seconde boucle dans la troisième boucle de procédé (3),
f) chauffer le mélange gazeux dans le second échangeur de chaleur (30) dans la troisième boucle de procédé à une température comprise entre 210 et 260°C,
g) introduire le mélange gazeux mis en température dans le réacteur de synthèse de méthanol (26) et convertir partiellement le troisième mélange en méthanol et en eau,
h) reconduire le troisième mélange de méthanol et d'eau à travers le second échangeur de chaleur (30) dans le premier étage séparateur de phases liquide/gaz (22),
i) séparer les composants liquides du mélange de méthanol et d'eau dans le premier étage séparateur de phases liquide/gaz (22) et reconduire celui-ci par la seconde boucle de procédé (2) dans l'étage de désorption (6), et
j) dériver en continu un mélange d'eau et de méthanol de l'étage de désorption (6).

14. Procédé selon la revendication 13,
comprenant le passage du troisième mélange de méthanol et d'eau par une dérivation de fluide (31) dans la troisième boucle de procédé (3) entre le réacteur de synthèse de méthanol (26) et le second échangeur de chaleur (30),
une partie du troisième mélange de méthanol et d'eau étant déviée vers une sortie de fluide (38).

15. Procédé selon la revendication 14,
**caractérisé en ce que**
une partie du troisième mélange de méthanol et d'eau est reconduite de la sortie de fluide (38) par un retour de phase liquide (39) dans l'étage de désorption (6).

16. Procédé selon la revendication 15,
**caractérisé en ce que**
la partie du troisième mélange de méthanol et d'eau dans le retour de phase liquide (39) est comprimée par une pompe de transfert (40) et/ou est séparé de ses composants gazeux dans un second étage séparateur de phases (41) liquide/gaz.

17. Procédé selon l'une des revendications 13 à 16,
**caractérisé en ce que**
dans la première conduite de circulation (4) en amont de l'étage de désorption (6) et/ou en amont de l'étage d'absorption (5) on contrôle chaque fois la température ou la mise en température (34, 35) dans la première boucle de procédé (1).
